# FASCICULE DE BREVET EUROPEEN

(11) **EP 3 328 448 B1**
(45) Date de publication et mention de la délivrance du brevet: **03.07.2019**
(21) Numéro de dépôt: 16757310.4
(22) Date de dépôt: 29.07.2016
(51) Int. Cl.: A61L 9/12, A01M 1/20

(54) **DIFFUSEUR DE SUBSTANCE VOLATILE ATMOSPHERIQUE D'INTERIEUR AVEC UNE ZONE DE DÉCLENCHEMENT DÉFORMABLE**
INNENDIFFUSOR FÜR FLÜCHTIGE ATMOSPHÄRISCHER STOFFE MIT FORMÄNDERNDEM FREISETZUNGSBEREICH
INNER VOLATILE ATMOSPHERIC SUBSTANCE DIFFUSER WITH A SHAPE-CHANGING RELEASE AREA

(30) Priorité: 30.07.2015 FR 1557333
(43) Date de publication de la demande: 06.06.2018
(73) Titulaire: SAS Carestia, 06130 Grasse (FR)
(72) Inventeur: DEVASSINE, Mickael, Chattanooga, Tennessee 37421 (US); MARCELLI - QUERO, Marie-Hélène, 06480 La Colle sur Loup (FR)
(74) Mandataire: Derambure, Christian
(86) Numéro de dépôt international: PCT/FR2016/051978
(87) Numéro de publication internationale: WO 2017/017387

(56) Documents cités:
- JP-A- H0 385 180
- US-A- 5 334 361
- US-B1- 6 254 836
- US-B2- 8 277 940

## Description

L'invention concerne un diffuseur de substance volatile atmosphérique, d'intérieur, compact et portatif, substantiellement monobloc, apte à être agité sans préjudice, omni positionnable, prêt à l'emploi, utilisable à la demande par déclenchement manuel en fonction du besoin, à fonctionnement autonome, à diffusion durable, le cas échéant pour plusieurs utilisations et notamment pour des substances différentes, apte éventuellement à être commercialisé et gardé avant utilisation sans la nécessité d'une enveloppe extérieure étanche, jetable après usage.

Si dans une application particulière, la substance volatile est une fragrance, il peut s'agir d'une substance différente mais analogue au regard de sa diffusion, par exemple une substance ayant des propriétés insecticides ou répulsives d'animaux (tels que mites ou moustiques), cette liste n'étant pas limitative.

Une application d'un tel diffuseur de fragrance dans laquelle on tire un excellent parti de ses caractéristiques constructives et fonctionnelles et qui participe à la définition du domaine de l'invention, consiste à le placer dans l'habitacle d'un véhicule automobile où il est suspendu au rétroviseur du pare-brise avant grâce à un moyen de suspension. Cette application n'est pas exclusive d'autres, non limitatives, dans lesquelles un tel diffuseur est placé dans une pièce ou un local d'une construction notamment une habitation, ou bien dans une armoire ou une zone de rangement.

Plus spécialement, l'invention a pour objet un diffuseur de substance volatile (en particulier fragrance) atmosphérique, d'intérieur, une plaquette comportant plusieurs tels diffuseurs, une plaque spécialement destinée à constituer la plaque intérieure d'un tel diffuseur et, enfin, un procédé de fabrication d'un tel diffuseur.

On connaît de manière générale, nombre de dispositifs de désodorisation ou de rafraîchissement de l'atmosphère d'une pièce d'habitation ou d'un véhicule par exemple.

Ceux d'une première famille visent à absorber les mauvaises odeurs. L'invention ne fait pas partie de cette première famille.

D'autres, fort divers, visent à répandre une fragrance dans l'atmosphère.

Il peut s'agir de vaporisateurs de fragrance (par exemple des bombes aérosols), avec lesquels on répand une fragrance de façon quasi instantanée. Le document FR 2930460 décrit un vaporisateur de parfum qui comprend une poche souple déformable avec un orifice de distribution, associée à des moyens de ressort et d'armement pour initialiser la poche et faire varier son volume. La poche contient des microcapsules de fragrance appliquées sur une ou sur les deux parois. En écartant les parois avec les moyens ressort, les microcapsules de fragrance sont détachées et brisées et de l'air chargé de la fragrance va être vaporisé par l'orifice de distribution. L'invention ne fait pas non plus partie de la famille des vaporisateurs.

Au lieu de vaporisateurs, il peut s'agir de diffuseurs de fragrance atmosphérique, d'intérieur, c'est-à-dire, conformément à l'acception courante, un dispositif permettant à une fragrance de s'évaporer lentement, de sorte à se répandre et à se propager dans l'atmosphère avoisinante plus ou moins dans toutes les directions et plus ou moins uniformément.

Leurs réalisations sont diverses quant à la réalisation ou le fonctionnement.

Dans certaines réalisations, le fonctionnement nécessite une alimentation électrique, des ultrasons, une flamme ou une combustion (par exemple bougies parfumées, papier d'Arménie).

Dans certaines réalisations, le diffuseur doit être « rechargé » au fur et à mesure de son utilisation par exemple avec des gouttes d'huiles essentielles déposées sur un support.

Dans certaines réalisations, le diffuseur comprend un flacon contenant un liquide parfumé et des mèches.

Dans certaines réalisations (voir par exemple US 4905898), le diffuseur - par exemple un pot-pourri, une pomme de senteur, un pochon odorant - comprend des fleurs ou des feuilles séchées (par exemple de lavande) ou des rameaux, des écorces ou des peaux de plantes odoriférantes (par exemple de thym, de romarin, de menthe, d'eucalyptus, d'orange), disposés dans un réceptacle tel qu'un vase ou un sachet en tissu.

Aucune de ces réalisations ne combine toutes les exigences d'un diffuseur de fragrance atmosphérique, d'intérieur conforme au domaine de l'invention.

Le document US 5071704 décrit un diffuseur qui comprend une couche poreuse incorporant une fragrance. Il comprend ensuite, sur la première face de la couche poreuse, une membrane de limitation du taux de diffusion de la fragrance et sur la face de cette membrane, une couche décorative. Il comprend enfin, sur la seconde face de la couche poreuse, une couche imperméable et sur la face de cette couche imperméable, une couche adhésive. L'utilisateur peut exercer une pression sur ce dispositif pour délivrer la fragrance.

Le document WO 2011/097605 décrit un composite pouvant être utilisé comme rafraîchisseur d'air. Il comprend une couche de mousse imbibée de fragrance par passage dans un bain de contenant la fragrance. La fragrance est délivrée à la demande suite à une pression exercée sur la couche de mousse. Il comprend également, du côté supérieur de la couche de mousse, une couche polymère imperméable à la fragrance, mais pourvue de perforations permettant d'expulser la fragrance à la demande et, du côté inférieur de la couche de mousse, une couche adhésive double face imperméable à la fragrance et enfin une couche inférieure.

Le document US 5334361 décrit un diffuseur qui comprend un corps poreux pourvu d'une cavité dans laquelle est insérée un tampon imprégné d'une fragrance. Dans une réalisation, le corps poreux comporte une partie renflée compressible de part et d'autre de laquelle est disposé le tampon, qui, par compression facilite la diffusion.

Une autre réalisation applicable principalement comme patch pour le corps mais aussi, éventuellement pour un véhicule automobile, est décrite dans le document JP2002345942 qui décrit un composite ayant une couche imprimée incluant des microparticules de fragrance rompues par pression et friction.

Ces réalisations ne présentent pas, parmi d'autres, l'exigence d'un diffuseur de fragrance atmosphérique, d'intérieur, conforme au domaine de l'invention, de permettre plusieurs utilisations distinctes séparées dans le temps et a fortiori pour des fragrances différentes.

Le document US 4889755 décrit non un diffuseur, mais un échantillonneur de parfum extractible qui comporte un support en deux parties superposées, dont une recevant des microcapsules de parfum ayant un diamètre compris entre 6 et 500 microns, préférentiellement entre 12 et 30 microns. Pour la mise en oeuvre, on déplace les deux parties l'une par rapport à l'autre dans leur plan. Par ce mouvement, on frotte les microcapsules qui se cassent et libèrent le parfum. Avec un tel dispositif, tout le parfum est libéré en une fois et sans contrôle.

Les documents US 8277940 et US 2013/0078421 ne décrivent pas des diffuseurs tels que celui du domaine de l'invention, mais des patchs ou des bandages destinés à être appliqués sur la peau et pouvant émettre une fragrance, grâce à des microcapsules rompues par compression. Le document US 2009/0313865 ne décrit pas non plus de tels diffuseurs, mais des cartes de voeux, des enveloppes et des sacs délivrant une fragrance à l'ouverture, grâce à la prévision de microcapsules de fragrance insérées entre deux couches de mousse disposées l'une contre l'autre, qui, pour le fonctionnement, sont écartées l'une de l'autre par l'utilisateur de sorte à rompre les microcapsules.

Le document US 4254179 ne décrit pas non plus un diffuseur tel que celui du domaine de l'invention, mais une couche de mousse imprégnée d'une fragrance masquant la mauvaise odeur de la mousse comme le polyuréthane, utilisée pour la fabrication de moquette. A cet effet, il est prévu d'incorporer au sein même dans la mousse des microcapsules de fragrance fines (74 microns ou moins) pour faciliter leur dispersion dans la mousse.

Un diffuseur de fragrance pour véhicule automobile a été conçu par Julius Sämann vers 1950. Il consiste en une plaque absorbante imprégnée d'une fragrance volatile et dont le contour représente un sapin, qui, jusqu'à utilisation est enfermée dans une enveloppe imperméable plate. Une cordelette en boucle traverse un trou prévu à une extrémité de la plaque. Pour la mise en oeuvre, l'utilisateur ouvre l'enveloppe en la déchirant, en extrait totalement la plaque, et jette l'enveloppe. Ensuite, il accroche la boucle de la cordelette au rétroviseur du pare-brise avant du véhicule automobile dont il souhaite parfumer l'habitacle. La plaque est alors suspendue au rétroviseur et la fragrance qui l'imprégnait diffuse dans l'atmosphère, à intérieur de l'habitacle. Lorsque l'utilisateur l'estime nécessaire, parce que par exemple la diffusion de la fragrance lui apparaît terminée ou devenue inefficace, il décroche la boucle de la cordelette du rétroviseur, enlève la plaque en forme de sapin et la jette.

Le document US 2757957 propose trois perfectionnements en vue de résoudre trois problèmes. Le premier problème est que, lors de l'ouverture, l'utilisateur touche la plaque imprégnée de fragrance, laquelle est huileuse ou collante. Pour solutionner ce premier problème, il est prévu la cordelette de préhension en forme de boucle précédemment mentionnée et, en outre, qu'une partie de la cordelette soit écartée de la plaque imprégnée de fragrance et aisément accessible par l'utilisateur, une fois l'enveloppe ouverte. Le deuxième problème est que le diffuseur devrait pouvoir rester opérant pendant une longue durée, ce qui ne peut être le cas si la plaque est extraite en totalité de l'enveloppe. Pour solutionner ce deuxième problème, il est prévu de n'extraire la plaque imprégnée de fragrance de l'enveloppe qu'en partie, et ce de plus en plus en fonction du besoin. Ainsi, la partie de la plaque imprégnée de fragrance située hors de l'enveloppe assure la fonction de diffusion de la fragrance, tandis que la partie restée dans l'enveloppe est censée ne pas diffuser la fragrance qui s'y trouve. Le troisième problème est que l'enveloppe est difficile à déchirer à ses extrémités solidarisées. Pour solutionner ce troisième problème, il est prévu des amorces de déchirure dans l'une des extrémités solidarisées. Celles-ci, judicieusement écartées transversalement, remplissent en outre une fonction de maintien de la plaque imprégnée de fragrance pour partie hors de de l'enveloppe et pour partie à l'intérieur de celle-ci.

Le document US 3065915 décrit un perfectionnement au diffuseur du document US 2757957 en vue de résoudre un autre problème qui découle de la solution au deuxième problème. Cet autre problème est que l'utilisateur ne sait dans quelle mesure la plaque imprégnée de fragrance est située dans l'enveloppe en fonction des besoins. Pour solutionner cet autre problème, il est prévu que l'enveloppe, opaque, comporte une fenêtre longitudinale à travers laquelle l'utilisateur peut voir la feuille absorbante pourvue d'un marquage visible correspondant à une durée d'utilisation.

Le diffuseur de fragrance pour véhicule conçu par Julius Sämann est commercialisé à travers le monde, à grande échelle, sous les marques MAGIC TREE, LITTLE TREE, ARBRE MAGIQUE, WUNDER-BAUM, selon les pays. Les diffuseurs représentés sur le site Internet https://www.car-freshner.com ne semblent pas comporter les quatre perfectionnements précédemment exposés (voir documents US 2757957 et US 3065915).

S'agissant de son but, l'invention se rattache à cette famille de diffuseurs.

Le document US 2013/0056549 décrit un autre perfectionnement au diffuseur précédemment décrit en vue de résoudre un autre problème qui est que la plaque imprégnée de fragrance est rugueuse, ce qui ne permet pas une impression fine. Pour solutionner cet autre problème, il est prévu une autre plaque supplémentaire - à savoir une plaque d'impression - disposée parallèlement à - et en regard de - la plaque imprégnée de fragrance, grâce à un clip portant ces deux plaques.

Le document US 7926735 décrit une variante de réalisation au diffuseur précédemment décrit en vue de résoudre le premier problème précédemment mentionné (à savoir que lors de l'ouverture, l'utilisateur touche la plaque imprégnée de fragrance). Pour solutionner ce problème, il est prévu qu'une partie de la paroi de l'enveloppe forme un volet de ventilation détachable, qui, avant d'être détaché au moyen d'une languette de préhension pour permettre la diffusion de la fragrance par l'ouverture de ventilation correspondante, est maintenu en position fermée, de façon hermétique. Le document US 5383598 prévoit également que l'enveloppe dans laquelle est enfermée la plaque imprégnée de fragrance comporte des ouvertures de ventilation. Le document US 2979268 décrit une réalisation proche, avec des parties détachables situées dans les angles de l'enveloppe et des canaux de ventilation entre la plaque imprégnée de fragrance et l'enveloppe.

Le document US 6557778 vise également à résoudre le premier problème précédemment mentionné et prévoit que la partie d'extrémité supérieure de la plaque imprégnée de fragrance soit fixée à demeure à la partie d'extrémité de l'enveloppe qui est pourvue, en dessous, d'une ligne frangible.

Le document US 8 277 940 décrit un patch ou un bandage destiné à émettre une senteur sur commande, comprenant des réservoirs compressibles contenant une substance volatile. Il n'est pas prévu la présence d'un matériau poreux.

Le document US 6 254 836 décrit un rafraichisseur d'air émetteur de senteur comprenant une sphère compressible de substance volatile sous forme de gaz en un matériel absorbant. Il n'est prévu qu'une paroi extérieure perméable.

Il apparaît que les diffuseurs de fragrances connus de la famille à laquelle se rattache l'invention soulèvent nombre de problèmes, présentent nombre d'inconvénients et comportent nombre de limites, dont ceux exposés précédemment ne sont pas exclusifs d'autres. Par exemple, du fait de leur conception, ces diffuseurs ne peuvent diffuser qu'une seule fragrance, ils ne permettent pas plusieurs utilisations distinctes séparées dans le temps, et ils nécessitent impérieusement d'être gardés avant utilisation dans une enveloppe extérieure étanche.

L'invention vise à résoudre ces problèmes, à surmonter ces inconvénients et à dépasser ces limites. Plus précisément, elle propose un diffuseur de substance volatile (en particulier fragrance) atmosphérique, d'intérieur, de la famille précédemment décrite, qui répond aux exigences suivantes, plus spécialement exposées pour un diffuseur de fragrance:
- Être compact, et portatif, ce qui signifie ici une dimension maximale de l'ordre d'une quinzaine, plus particulièrement une dizaine de centimètres, un poids maximum de quelques grammes.
- Être substantiellement monobloc, ce qui signifie ici pouvant être appréhendé comme un tout substantiellement unitaire et solidaire et non pas être morcelé en plusieurs parties séparées les unes des autres.
- Être apte à être agité de façon plus ou moins importante, comme c'est le cas pour un diffuseur de fragrance suspendu au rétroviseur d'un véhicule.
- Être omni positionnable, ce qui signifie ici que le diffuseur peut être disposé dans n'importe quelle position, sans préjudice pour son fonctionnement, et cela contrairement par exemple à un diffuseur comportant un média liquide parfumé dans un contenant ouvert. La capacité à être omni positionnable n'exclut pas une position normale d'utilisation (notamment le diffuseur suspendu).
- Être prêt à l'emploi, ce qui signifie ici que le diffuseur ne nécessite pas une préparation autre que - s'il est prévu un emballage -, l'ouverture de celui-ci, l'extraction du diffuseur et sa mise en position. En d'autres termes, il n'est pas nécessaire, par exemple, de charger le diffuseur en fragrance, ou d'organiser ses parties constitutives.
- Être utilisable à la demande par déclenchement manuel en fonction du besoin ce qui signifie ici, en premier lieu, que le déclenchement du fonctionnement du diffuseur ne résulte que d'une action manuelle et non d'une action d'une autre nature comme électrique, chaleur... et, en second lieu, que ce déclenchement peut intervenir à tout moment dès lors que l'utilisateur le décide.
- Être à fonctionnement autonome ce qui signifie ici que le diffuseur, une fois son fonctionnement déclenché, fonctionne de lui-même, sans la nécessité par exemple d'une énergie électrique, d'ultrasons, de flammes ou de combustion.
- Être à diffusion durable, notamment pour plusieurs utilisations ce qui signifie ici que dans les conditions normales de mise en oeuvre, la diffusion, une fois déclenchée, se poursuit continûment dans le temps, pendant une durée significative, par exemple de l'ordre d'au moins un mois, voire plus.
- Permettre le cas échéant plusieurs utilisations ce qui signifie ici qu'un diffuseur donné peut, moyennant bien entendu un déclenchement manuel de l'utilisateur à chaque fois, être utilisé plusieurs fois dans le temps, non seulement de façon consécutive mais aussi de façon espacée (avec une ou plusieurs périodes intermédiaires pendant lesquelles le diffuseur n'est pas actif ou n'est que très peu actif) ; ce que ne permet pas un diffuseur tel que celui commercialisé sous les marques MAGIC TREE, LITTLE TREE, ARBRE MAGIQUE, WUNDER-BAUM ou décrit dans les documents US 2757957, US 3065915, US 2013/0056549 et autres. Etant donné que « qui peut le plus peut le moins », le diffuseur peut être prévu pour une utilisation unique, c'est-à-dire une seule diffusion.
- Permettre à l'utilisateur de savoir, dans le cas d'un diffuseur pour plusieurs utilisations consécutives ou espacées, dans quelle mesure il a été, ou est, mis en oeuvre et surtout peut encore l'être à l'avenir, et cela par un moyen plus efficient que celui décrit dans les documents US 2757957 et US 3065915.
- Être apte éventuellement à être commercialisé et gardé avant utilisation sans la nécessité d'une enveloppe extérieure étanche, ce qui n'exclut pas qu'il soit prévu une enveloppe d'emballage extérieure. Mais l'existence d'une telle enveloppe n'est pas une condition indispensable.
- Être jetable après usage ce qui signifie ici que le diffuseur n'a pas à être rechargé ou qu'une partie constitutive n'a pas à être gardée.
- Éviter que lors de la mise en oeuvre du diffuseur, l'utilisateur ne soit en contact avec la plaque imprégnée de fragrance, et cela par un moyen plus efficient que celui décrit dans les documents US 2757957, US 3065915, US 7926735, US 2979268 et US 6557778.
- Permettre une mise en oeuvre aisée, notamment en évitant des opérations d'arrachage difficiles ou conduisant à un résultat inesthétique, comme tel est le cas avec les diffuseurs décrits dans les documents US 2757957, US 3065915 et US 6557778. Éviter que l'enveloppe extérieure du diffuseur - si elle est prévue -, ne reste accrochée en totalité ou en partie à la plaque imprégnée de fragrance, tel que cela est décrit dans les documents US 2757957 et US 3065915.
- Éviter la présence d'ouvertures, de volets de fermeture, de parties détachables, etc., comme tel est le cas avec les diffuseurs décrits dans les documents US 7926735, US 5383598 et US 2979268.
- Permettre une impression fine de qualité, en quadrichromie, ou teintes directes résistantes aux composants du média liquide, et, directement, sans la nécessité d'un composant supplémentaire à cette fin, comme tel est le cas avec le diffuseur décrit dans le document US 2013/0056549.
- Être apte à être placé dans l'habitacle d'un véhicule automobile, tout spécialement pour être suspendu, par exemple au rétroviseur du pare-brise avant.
- Être d'une fabrication aisément mécanisable et être peu coûteux.

Telles sont les exigences que l'invention vise à satisfaire et, corrélativement, les problèmes, les inconvénients et les limites qu'elle vise, respectivement, à résoudre, à surmonter et à dépasser.

Ci-après, un exposé de l'invention telle que caractérisée dans les revendications.

Selon un premier aspect, l'invention a pour objet un diffuseur de substance volatile (en particulier fragrance) atmosphérique, d'intérieur, comportant :
- deux parois extérieures, ayant chacune une face extérieure et une face intérieure, espacées l'une en regard de l'autre, parallèlement,
   ∘ une étant perméable à la substance volatile, en direction transversale, de part en part,
   ∘ une pouvant, dans au moins une zone de déclenchement déformable, être déformée en direction transversale et dans le sens extérieur-intérieur consécutivement à une pression de déclenchement, suffisante, de mêmes direction et sens, exercée manuellement par l'utilisateur du diffuseur pour déclencher la diffusion,
- une plaque intérieure disposée entre les deux parois extérieures, en regard d'elles, adjacente à la face intérieure de la paroi extérieure perméable, formant avec les deux parois extérieures un ensemble solidaire,
   ∘ pourvue d'interstices communicants et ouvrants, et compressible en direction transversale,
   o ménageant en elle au moins un logement au droit d'une zone de déclenchement déformable, recevant et à maintenant en position une capsule de substance volatile,

- au moins une capsule de substance volatile, unitaire ou formée de l'agrégat de plusieurs capsules élémentaires,
   o qui, ayant son intégrité, enferme une substance volatile, et est adjacente à la plaque intérieure et à la face intérieure de la paroi extérieure perméable,
   o qui est apte à être rompue moyennant une pression transversale suffisante correspondant à la pression de déclenchement exercée par l'utilisateur, en libérant ainsi la substance volatile,
de sorte que :
- avant mise en oeuvre, la capsule de substance volatile a son intégrité,
- pour déclencher la diffusion de la substance volatile d'une capsule de substance volatile, et donc la mise en oeuvre du diffuseur, l'utilisateur exerce la pression de déclenchement sur la zone de déclenchement déformable correspondant à la capsule, ce qui rompt cette capsule et libère la substance volatile qui diffuse vers la face intérieure de la paroi extérieure perméable, d'une part directement, d'autre part indirectement via les interstices communicants et ouvrants de la plaque intérieure, puis diffuse à travers la paroi extérieure perméable et ensuite dans l'atmosphère environnante.

Selon une réalisation, avant toute première utilisation, ni les parois extérieures ni la plaque intérieure ne sont substantiellement imprégnées de la substance volatile, celle-ci étant enfermée dans les capsules de substance volatile.

Selon une réalisation, le diffuseur comporte une pluralité de zones de déclenchement déformables analogues, , une pluralité de logements analogues, une pluralité de capsules de substance volatile de dimensions analogues, reçues et maintenues en position dans la pluralité de logements, et des espaces entre les zones de déclenchement déformables et entre les logements dans lesquels il n'y a pas de capsule de substance volatile, les capsules de substance volatile étant distantes et séparées les unes des autres de sorte qu'une capsule de substance volatile peut être rompue sans que soient rompues toutes les autres capsules de substance volatile du diffuseur, l'utilisateur ayant le choix de déclencher la diffusion d'une capsule de substance volatile sans déclencher celle de toutes les autres capsules de substance volatile, le diffuseur permettant ainsi plusieurs utilisations distinctes, consécutives ou espacées.

Selon une réalisation, une capsule de substance volatile a une dimension supérieure à 3 mm, notamment de l'ordre de 5 mm, et dans lequel les zones de déclenchement déformables et les logements sont espacés les uns des autres d'une distance au moins égale à de l'ordre de plusieurs fois la dimension d'une capsule de substance volatile, notamment plus deux à trois fois.

Selon une réalisation, les capsules de substance volatile comportent la même substance volatile. Selon une autre réalisation, elles comportent des substances volatiles différentes.

Selon une réalisation, une même paroi extérieure est perméable et comporte la au moins une zone de déclenchement déformable.

Selon une réalisation, les deux parois extérieures sont perméables et/ou comportent la au moins une zone de déclenchement déformable.

Selon une réalisation, les deux parois extérieures sont analogues, le diffuseur étant symétrique.

Selon une réalisation, une capsule de substance volatile ayant son intégrité est adjacente à la face intérieure des deux parois extérieures.

Selon une réalisation, une capsule de substance volatile ayant son intégrité est au contact de la face intérieure de la paroi extérieure perméable.

Selon une réalisation, la paroi extérieure perméable, globalement plate, comporte au moins une zone conformée en forme générale de calotte sphérique à concavité tournée vers l'intérieur, saillant de la face extérieure de la paroi extérieure perméable, de forme globalement complémentaire de la partie de la capsule de substance volatile ayant son intégrité au contact de la face intérieure de cette paroi extérieure perméable.

Selon une réalisation, la au moins une zone conformée saille de la face extérieure de la paroi extérieure perméable, de l'ordre du millimètre ou de quelques millimètres.

Selon une réalisation, une paroi extérieure comportant la au moins une zone conformée est en papier ou en carton et la au moins une zone conformée est réalisée par gaufrage.

Selon une réalisation, une zone conformée et une zone de déclenchement déformable sont pour partie au moins communes.

Selon une réalisation, le diffuseur comporte autant de zones conformées que de zones de déclenchement déformables.

Selon une réalisation, l'une ou les deux parois extérieures sont en papier ou carton d'un grammage compris entre 250 grammes et 450 grammes, plus particulièrement entre 350 grammes et 400 grammes.

Selon une réalisation, le diffuseur comporte vers, ou sur, la face extérieure d'au moins une paroi extérieure, notamment la paroi extérieure comportant la au moins une zone de déclenchement déformable, au moins un moyen de repérage de la localisation de ladite au moins une zone de déclenchement déformable.

Selon une réalisation, le moyen de repérage est la saillie sur la face extérieure de la paroi extérieure perméable constituée par la zone conformée correspondante.

Selon une réalisation, la plaque intérieure est réalisée sous la forme de matière plastique en mousse à cellules ouvertes, ou sous la forme de non-tissé, ou moyennant une structure ayant pour effet de retarder le temps mis par la substance volatile pour migrer de la capsule une fois rompue à la paroi extérieure perméable..

Selon une réalisation, la plaque intérieure a, en l'absence de toute sollicitation de compression, une épaisseur légèrement plus petite que la dimension d'une capsule de substance volatile.

Selon une réalisation, la plaque intérieure a, en l'absence de toute sollicitation de compression, une épaisseur plus petite que la dimension d'une capsule de substance volatile, de l'ordre du millimètre ou d'une fraction de millimètre.

Selon une réalisation, la plaque intérieure est adjacente aux deux faces intérieures des parois extérieures.

Selon une réalisation, la plaque intérieure est au contact de la face intérieure de la paroi extérieure perméable.

Selon une réalisation, la plaque intérieure est compressible substantiellement élastiquement.

Selon une réalisation, les deux parois extérieures et la plaque intérieure forment un ensemble solidaire par collage.

Selon une réalisation, un logement de capsule est un espace vide, débouchant par une ouverture vers une paroi extérieure perméable, dont la dimension dans le plan de la plaque intérieure est ajustée à la dimension de la capsule de substance volatile dans ce même plan, de sorte que la capsule de substance volatile soit maintenue en position.

Selon une réalisation, un logement de capsule est un trou traversant.

Selon une réalisation, les deux parois extérieures sont distinctes l'une de l'autre.

Selon une réalisation, les deux parois extérieures sont deux parties d'une même pièce pliée à l'endroit de deux rainages, une portion du champ périphérique de la plaque intérieure étant recouverte par une troisième partie de la pièce entre les deux rainages.

Selon les réalisations, le champ périphérique de la plaque intérieure est soit libre et exposé à l'atmosphère environnante, soit pour partie recouvert, soit totalement recouvert.

Selon une réalisation, une face extérieure au moins des parois extérieures comporte une impression, des lettres, chiffres, motifs.

Selon une réalisation, le diffuseur comporte en outre au moins une partie d'un moyen de suspension, notamment un trou traversant apte à recevoir un lien d'accrochage.

Selon une réalisation, le diffuseur se suffit à lui-même pour la fonction de diffusion et ne comporte pas d'enveloppe extérieure. Selon une autre réalisation, le diffuseur se suffit à lui-même pour la fonction de diffusion et comporte une enveloppe extérieure dans laquelle il est placé.

Selon une réalisation, le diffuseur est caractérisé en ce que la substance volatile est une fragrance ; le diffuseur étant alors un diffuseur de fragrance.

Selon un deuxième aspect, l'invention a pour objet une plaquette comportant plusieurs diffuseurs de substance volatile atmosphérique, d'intérieur, tels qu'ils ont été décrits précédemment, disposés côte à côte et séparés par des lignes frangibles.

Selon un troisième aspect, l'invention a pour objet une plaque, spécialement destinée à constituer la plaque intérieure d'un diffuseur de substance volatile atmosphérique, d'intérieur tel qu'il a été précédemment décrit, qui est pourvue d'interstices communicants et ouvrants, qui est compressible en direction transversale, et qui ménage en elle au moins un logement apte à recevoir et maintenir en position une capsule de substance volatile.

Selon un quatrième aspect, l'invention a pour objet un procédé de fabrication d'un diffuseur de substance volatile atmosphérique, d'intérieur, tel qu'il a été précédemment décrit, dans lequel :
- on a à disposition deux parois extérieures ou une pièce pourvue de rainages permettant de réaliser les deux parois extérieures,
- on a à disposition une plaque intérieure pourvue d'interstices communicants et ouvrants, compressible en direction transversale, et ménageant en elle au moins un logement apte à recevoir et maintenir en position une capsule de substance volatile,
- on a à disposition au moins une capsule de substance volatile, unitaire ou formée de l'agrégat de plusieurs capsules élémentaires,
- on dispose la plaque intérieure sur la face intérieure d'une première paroi extérieure, en regard de cette première paroi extérieure et on les solidarise,
- alors que la au moins une capsule de substance volatile est reçue dans le au moins un logement de la plaque intérieure, on dispose la face intérieure d'une seconde paroi extérieure sur la plaque intérieure, en regard de la plaque intérieure et on les solidarise.

On décrit maintenant brièvement les figures des dessins.

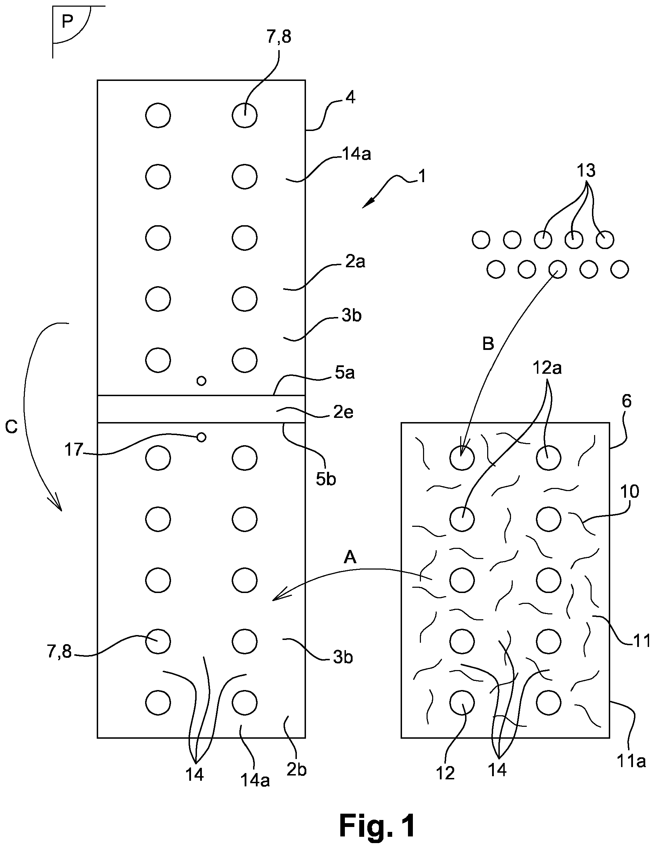

La figure 1 est une vue en plan d'éléments entrant dans la réalisation d'un mode particulier de réalisation d'un diffuseur de substance volatile atmosphérique, d'intérieur, conforme à l'invention, ici un diffuseur de fragrance, à savoir un diffuseur pour plusieurs utilisations, lesdits éléments étant une pièce rainée en papier permettant de former deux parois extérieures dont la figure 1 représente les faces intérieures, une plaque intérieure avec une pluralité de logements et une même pluralité de capsules de substance volatile (ici fragrance), unitaires, avec leur intégrité, ces éléments étant ici dissociés les uns des autres, avant d'être assemblés pour former le diffuseur.

La figure 2 est une vue en plan du diffuseur assemblé à partir des éléments représentés sur la figure 1, avant toute première utilisation.

Les figures 3 et 4 sont deux vues du diffuseur assemblé de la figure 1, respectivement d'extrémité et de côté.

Les figures 5A et 5B sont deux vues en coupe par un plan transversal, du diffuseur représenté sur les figures 2 à 4, selon les lignes 5A-5A et 5B-5B de la figure 1, pour la figure 5A, au droit de capsules de fragrance et pour la figure 5B dans l'espace entre les capsules de fragrance.

La figure 6 est une vue partielle, à plus grande échelle, d'une partie de la figure 5A, illustrant une capsule de fragrance, et plus généralement le diffuseur, avant déclenchement de la diffusion.

La figure 7 est une vue analogue à la figure 6, illustrant une capsule de fragrance, et plus généralement le diffuseur, après déclenchement de la diffusion.

Ci-après, et de référence aux dessins, un exposé détaillé d'un mode particulier de réalisation de l'invention dans lequel, il est prévu une pluralité de zones de déclenchement déformables analogues, une même pluralité de logements analogues, et une même pluralité de capsules de substance volatile (ici fragrance) de dimensions analogues et plusieurs espaces entre les zones de déclenchement déformables et les logements, dans lesquels il n'y a pas de capsule de fragrance (zones de déclenchement déformables, logements, capsules de fragrance sur lesquels on reviendra), cette réalisation permettant plusieurs utilisations du diffuseur, qui, moyennant un déclenchement manuel de l'utilisateur à chaque fois, peut être utilisé plusieurs fois dans le temps, tant de façon consécutive que de façon espacée. Par suite, ce mode de réalisation est appelé par convention « diffuseur pour plusieurs utilisations ». Ce mode particulier de réalisation n'est pas limitatif. Il peut, d'une part, être généralisé, d'autre part, faire l'objet de variantes. En particulier dans un autre mode de réalisation, appelé par convention « diffuseur pour une seule utilisation », il est prévu une seule zone de déclenchement déformable, un seul logement et une seule capsule de fragrance.

Un diffuseur de substance volatile (ici fragrance) atmosphérique, d'intérieur 1 (désigné parfois plus simplement « diffuseur »), dans sa réalisation diffuseur pour plusieurs applications, est tout spécialement destiné à être placé dans l'habitacle d'un véhicule automobile où il est suspendu au rétroviseur du pare-brise avant grâce à un moyen de suspension, dont une partie peut être intégrée au diffuseur 1 lui-même. Cette application n'est toutefois pas exclusive. D'autres, elles-mêmes non limitatives, dans lesquelles le diffuseur 1, pour plusieurs utilisations ou pour une seule utilisation, est destiné à être placé dans une pièce ou un local d'une construction notamment une habitation, ou bien dans une armoire ou une zone de rangement, ou analogue peuvent être envisagées.

Le diffuseur 1, une fois assemblé, comporte d'abord, deux parois extérieures 2a, 2b, ayant chacune une face extérieure 3a et une face intérieure 3b, espacées l'une en regard de l'autre, sensiblement parallèlement.

Les deux parois 2a, 2b sont qualifiées d'extérieures, pour la raison qu'elles délimitent le diffuseur 1 vers l'extérieur de lui-même et dans son propre plan. La face 3a est qualifiée d'extérieure car elle forme la face visible de l'extérieur du diffuseur 1, et est en contact avec l'atmosphère avoisinante dans laquelle on souhaite que le diffuseur 1 diffuse sa fragrance. La face 3b est qualifiée d'intérieure 3b, car elle est se trouve à l'intérieur même du diffuseur 1 et, une fois le diffuseur 1 assemblé, est masquée à la vue.

Dans la réalisation de la figure 1, les deux parois extérieures 2a, 2b, sont deux parties analogues d'une même pièce 4, pliée à l'endroit de deux rainages 5a, 5b, ménageant entre eux une troisième partie 2c, qui recouvre une portion du champ périphérique d'une plaque intérieure 6 sur laquelle on reviendra. Par suite, les deux parois extérieures 2a, 2b sont analogues, présentent les mêmes caractéristiques, quant à la perméabilité à la fragrance, à la présence de zones de déclenchement déformables 7, de zones conformées 8, et de moyens de repérage 9, sur lesquels on reviendra. Le diffuseur 1 est alors symétrique.

Dans la réalisation représentée sur les figures, mais qui n'est pas exclusive d'autres, une même paroi extérieure 2a ou 2b est perméable à la fragrance et comporte les zones de déclenchement déformables 7, les zones conformées 8, et les moyens de repérage 9. Plus précisément, les deux parois extérieures 2a et 2b sont perméables à la fragrance et comportent les zones de déclenchement déformables 7, les zones conformées 8, et les moyens de repérage 9.

Dans une autre réalisation, les deux parois extérieures 2a, 2b sont distinctes l'une de l'autre. Dans ce cas, les deux parois extérieures 2a, 2b peuvent, soit présenter les mêmes caractéristiques, le diffuseur 1 étant symétrique, soit présenter des caractéristiques différentes. Par exemple, l'une des parois extérieures 2a, 2b peut être perméable à la fragrance et l'autre 2b, 2a, imperméable. Ou bien, l'une des parois extérieures 2a, 2b peut comporter des zones de déclenchement déformables 7, des zones conformées 8, et des moyens de repérage 9, tandis que l'autre des parois extérieures 2b, 2a n'en comporte pas. Dans ces réalisations, le diffuseur 1 n'est pas symétrique.

Dans la réalisation représentée, la pièce 4 de forme rectangulaire, est réalisée en papier ou carton d'un grammage compris entre 250 grammes et 450 grammes, plus particulièrement entre environ 350 grammes et 400 grammes, et d'une épaisseur de l'ordre du millimètre. Cette pièce 4 présente une longueur de l'ordre de 17,5 cm et une largeur de l'ordre de 5,5 cm, tandis que les deux parois extérieures 2a, 2b, et donc le diffuseur 1, de forme rectangulaire également, présente une longueur de l'ordre de 8,5 cm, une largeur de l'ordre de 5,5 cm, et une épaisseur hors tout de l'ordre de 0,5 cm. La forme rectangulaire n'est pas limitative. Les dimensions indiquées ne sont pas non plus limitatives, pourvu que le diffuseur 1 soit compact et portatif, adapté à sa destination, avec une dimension maximale de l'ordre d'une quinzaine, plus particulièrement une dizaine de centimètres et un poids maximum de quelques grammes.

Avec cette réalisation, les deux parois extérieures 2a, 2b présentent une tenue d'ensemble qui les rend en quelque sorte autoportantes, en étant globalement plates et rigides, ce qui n'exclut pas une capacité de déformation locale, comme on le verra. Les deux parois extérieures 2a, 2b définissent un plan de référence qualifié de plan P du diffuseur 1. Une direction au moins sensiblement perpendiculaire au plan P, comme la direction Q, est qualifiée de « transversale ».

Dans la réalisation représentée, chaque paroi extérieure 2a, 2b est perméable à la fragrance, en direction transversale Q, de part en part de la paroi, au moins dans le sens intérieur-extérieur allant de la face intérieure 3b à la face intérieure 3a de cette paroi.

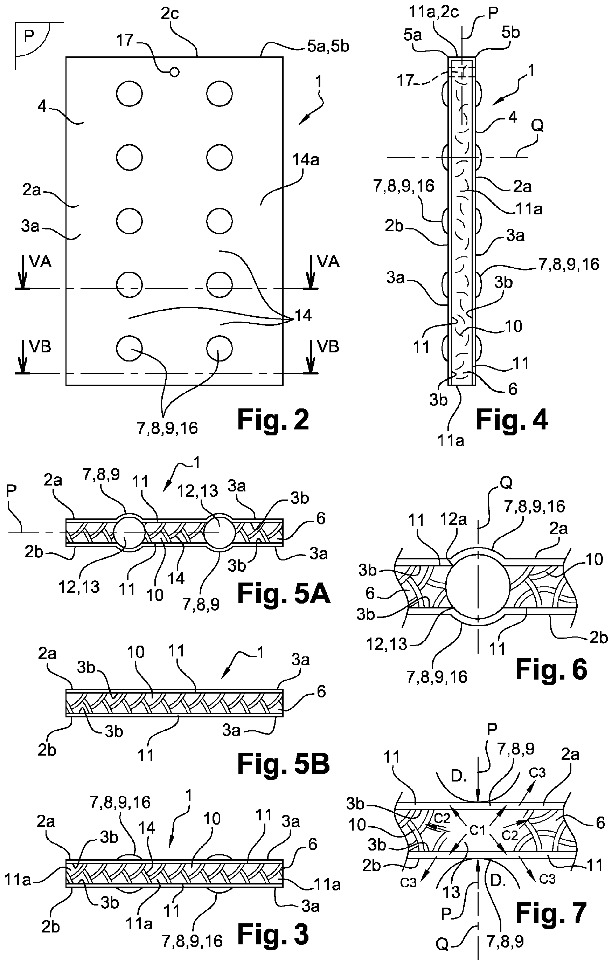

Dans cette réalisation, et pour un diffuseur pour plusieurs utilisations, chaque paroi extérieure 2a, 2b peut, dans une pluralité de zones de déclenchement déformables 7, être déformée en direction transversale Q, et dans le sens extérieur-intérieur de cette paroi extérieure (c'est-à-dire vers l'intérieur du diffuseur 1), consécutivement à une pression de déclenchement p (flèches p sur la figure 7), suffisante, de même direction transversale et de même sens, exercée manuellement par l'utilisateur du diffuseur 1 pour déclencher la diffusion. Cette disposition constructive avec celles associées sur lesquelles on reviendra, permettent plusieurs utilisations du diffuseur 1, comme il a été déjà exposé.

Dans la réalisation d'un diffuseur 1 pour une seule utilisation, il est prévu une seule zone de déclenchement déformable 7.

Le diffuseur 1, une fois assemblé, comporte ensuite une plaque intérieure 6, limitée par deux faces 11, disposée entre les deux parois extérieures 2a, 2b, en regard d'elles, raison pour laquelle elle est qualifiée d'intérieur, même si tout ou partie de son champ périphérique 11a est visible de l'extérieur du diffuseur 1, et en contact avec l'atmosphère avoisinante (voir figure 4).

Le terme « plaque » n'est pas en soi limitatif. Il est utilisé ici parce que l'objet correspondant est de surface globalement sensiblement plane, peu épais, et délimité pour avoir une forme géométrique, par exemple rectangulaire, ce qui correspond à ce que l'on peut désigner avec le terme de « plaque ».

La plaque intérieure 6 est adjacente aux deux parois extérieures 2a, 2b, c'est-à-dire que ses deux faces 11 sont adjacentes aux faces intérieures 3b des deux parois extérieures 2a, 2b. Dans une réalisation, l'adjacence est le contact.

Etant possible que dans une réalisation une seulement des deux parois extérieures 2a, 2b soit perméable à la fragrance, la plaque intérieure 6 est, dans ce cas, adjacente à cette paroi extérieure perméable et donc à sa face intérieure.

La plaque intérieure 6 est pourvue d'interstices 10, communicants entre eux et ouvrants sur ses faces 11, cette structure de la plaque intérieure permettant à la fragrance libérée de circuler dans la plaque intérieure jusqu'à atteindre l'une de ses faces 11, ou ses deux faces 11.

La plaque intérieure 6 est compressible en direction transversale Q, notamment substantiellement élastiquement. Cette compressibilité est locale, ce qui signifie que si la plaque intérieure 6 est comprimée dans une zone, elle n'est pas comprimée à une distance de cette zone de l'ordre de, par exemple, 1,5 fois l'épaisseur de la plaque intérieure 6.

Dans une réalisation, la plaque intérieure 6 est réalisée sous la forme de matière plastique en mousse à cellules ouvertes. Dans une autre réalisation, elle est sous la forme d'un non-tissé. Plus généralement, elle peut être avec une structure ayant pour effet de retarder le temps mis par la substance volatile pour migrer de la capsule une fois rompue à la paroi extérieure perméable. Ces réalisations ne sont pas exclusives d'autres assurant, d'une part, la communication interne et vers l'extérieur comme avec des vides ou des cellules ouvertes, d'autre part, la compressibilité locale.

La plaque intérieure 6 ménage en elle, pour un diffuseur pour plusieurs utilisations, une pluralité de logements 12, chacun d'eux étant situé au droit de l'une des zones de déclenchement déformables 7.

Un tel logement 12 est conçu de manière telle qu'il est apte à recevoir et à maintenir en position, dans un emplacement déterminé, une capsule de fragrance 13. Telle est sa fonction.

Dans la réalisation d'un diffuseur 1 pour une seule utilisation, il est prévu une seule capsule de fragrance 13.

Un logement 12 de capsule de fragrance 13 est un espace vide, débouchant par une ouverture 12a vers une paroi extérieure perméable 2a, 2b. Sa dimension dans le plan du diffuseur, ici le plan de la plaque intérieure 6 est ajustée à la dimension de la capsule de fragrance 13 dans ce même plan, de sorte que la capsule de fragrance 13 soit maintenue en position dans un emplacement déterminé.

Selon les cas, l'espace vide est un trou réalisé comme tel dans la plaque intérieure 6 ou bien une sorte de cavité réalisée dans la plaque intérieure 6 en repoussant ses parties situées au voisinage immédiat de ce qui est destiné à former cette cavité. Le premier cas est bien adapté à une plaque intérieure 6 en mousse et le second à une plaque intérieure 6 en non-tissé.

Dans la réalisation représentée d'un diffuseur 1 dont les deux parois extérieures 2a, 2b, sont perméables, le logement 12 peut être un trou traversant avec deux ouvertures 12a.

Etant possible que dans une réalisation une seulement des deux parois extérieures 2a, 2b soit perméable à la fragrance, le logement 12 peut alors être un trou borgne avec une seule ouverture 12a.

Selon les réalisations, le champ périphérique 11a de la plaque intérieure 6 est soit libre et exposé à l'atmosphère environnante, soit pour partie recouvert, soit totalement recouvert auquel cas il n'est pas exposé à l'atmosphère environnante. Par exemple, l'une ou les deux parois extérieures 2a, 2b peuvent comporter des rabats latéraux, qui lors de la fabrication sont pliés à angle droit et fixés, de sorte à recouvrir le champ périphérique 11a de la plaque intérieure 6.

Dans le cas où les deux parois extérieures 2a, 2b, appartiennent à la pièce 4, pourvue de rainages 5a, 5b, une portion du champ périphérique 11a est recouverte par la troisième partie 2c de la pièce 4 située entre les deux rainages 5a, 5b.

Dans la réalisation représentée, la plaque intérieure 6 est continue et d'un seul tenant.

Mais il est envisageable de prévoir, dans d'autres réalisations, une plaque intérieure 6 formée de parties élémentaires juxtaposées. Selon les cas, ces parties élémentaires de plaque intérieure 6 sont en continuité entre elles ou bien elles sont séparées les unes des autres par des barrières intermédiaires imperméables aux fragrances, de sorte à pour circonscrire la circulation et la diffusion de la fragrance à la seule partie de plaque intérieure comportant la capsule de fragrance 13 qui est rompue.

Le diffuseur 1, pour plusieurs utilisations, une fois assemblé, comporte ensuite une pluralité de capsules de fragrance 13.

Une telle capsule de fragrance 13 qui a son intégrité enferme une fragrance, laquelle est donc confinée dans la capsule.

Une telle capsule de fragrance 13 a une forme générale sphérique.

Une telle capsule de fragrance 13 est apte à être rompue moyennant l'exercice sur elle-même d'une pression transversale (radiale) suffisante. La fragrance qu'elle enfermait et confinait est alors libérée à l'extérieur d'elle.

Par capsule, on entend soit une capsule unitaire, comme représenté sur les figures, soit l'agrégat formé de plusieurs capsules élémentaires plus petites, juxtaposées de façon compacte.

Dans la réalisation d'un diffuseur 1 pour une seule utilisation, il est prévu une seule capsule de fragrance 13.

Une capsule de fragrance 13, ayant son intégrité, est adjacente à la plaque intérieure 6 et aux faces intérieures 3b des deux parois extérieures perméables 2a, 2b. Dans une réalisation, l'adjacence est le contact.

Etant possible que dans une réalisation une seulement des deux parois extérieures 2a, 2b soit perméable à la fragrance, la capsule de fragrance 13 est, dans ce cas, adjacente à cette paroi extérieure perméable et donc à sa face intérieure 3b.

La pression transversale exercée sur une capsule de fragrance 13 suffisante pour la rompre correspond à la pression de déclenchement p exercée par l'utilisateur sur la zone de déclenchement déformable 7 correspondante.

Avant toute première utilisation et toute mise en oeuvre, ni les parois extérieures 2a, 2b, ni la plaque intérieure 6 ne sont substantiellement imprégnées de la fragrance, celle-ci étant enfermée dans les capsules de fragrance 13, lesquelles ont leur intégrité.

Le diffuseur 1 se suffit à lui-même pour la fonction de diffusion pour laquelle il est destiné. Par suite, il n'est pas impératif de prévoir une enveloppe extérieure dans laquelle il est placé. Toutefois, il peut être envisagé de prévoir une telle enveloppe, par exemple pour y placer un encart ou comme support de communication ou pour protéger une impression, des lettres, chiffres, motifs, etc. prévus sur la face extérieure 3a d'une ou des deux parois extérieures 2a, 2b.

Pour déclencher la diffusion de la fragrance d'une capsule de fragrance 13, et donc la mise en oeuvre du diffuseur 1, l'utilisateur exerce la pression de déclenchement p (flèches p sur la figure 7) sur la zone de déclenchement déformable 7 correspondant à la capsule 13 souhaitée, ce qui rompt cette capsule 13 et libère la fragrance. Cette pression de déclenchement p est exercée typiquement avec le doigt D, ou bien en pinçant le diffuseur 1 à l'endroit de la capsule de fragrance 13, entre les deux doigts D, comme représenté sur la figure 7.

La fragrance diffuse alors vers les faces intérieures 3b des deux parois extérieures perméables 2a, 2b, d'une part directement (flèches c1 figure 7), d'autre part indirectement (flèches c2 figure 7) via les interstices 10 de la plaque intérieure 6. Puis, la fragrance diffuse à travers les deux parois extérieures perméables 2a, 2b et ensuite, à partir de là, dans l'atmosphère environnante (flèches c3 figure 7).

Il a été constaté que ce double flux de diffusion, par voie directe et par voie indirecte, avait pour effet de régulariser la diffusion dans le temps.

Dans la mesure où le champ périphérique de la plaque intérieure 6 n'est pas occulté, la fragrance peut également être diffusée par là.

Si un diffuseur 1 comportant une pluralité de zones de déclenchement déformables 7, de logements 12, de capsules de fragrance 13 est conçu pour plusieurs utilisations, l'on ne peut exclure qu'un utilisateur exerce la pression de déclenchement p simultanément sur toutes les zones de déclenchement déformables 7, auquel cas, toutes les capsules de fragrance 13 seront rompues simultanément, le diffuseur 1 ne pouvant alors être utilisé qu'une seule fois.

Le diffuseur 1, pour plusieurs utilisations, comporte des espaces 14 entre les zones de déclenchement déformables 7 et entre les logements 12, dans lesquels il n'y a pas de capsules de fragrance 13. Ainsi, les capsules de fragrance 13 sont distantes et séparées les unes des autres. Ce faisant, il est possible de rompre une capsule de fragrance 13 sans rompre toutes les autres capsules de fragrance 13 du diffuseur 1. L'utilisateur du diffuseur 1 a donc le choix de déclencher la diffusion d'une capsule de fragrance 13, sans déclencher celle des autres. Et c'est ainsi que le diffuseur 1 permet pour plusieurs utilisations distinctes, consécutives ou espacées.

Une capsule de fragrance 13 a pour caractéristique d'avoir une taille substantielle de sorte à pouvoir être individualisée et d'avoir un emplacement déterminé et identifiable. Par-là, on entend qu'une capsule de fragrance 13 a une dimension supérieure à 3 mm, notamment de l'ordre de 5 mm. Ce faisant, les capsules de fragrance 13 du diffuseur 1 ne peuvent être qualifiées de « microcapsules ». Tout au contraire, elles pourraient éventuellement être qualifiées de « macrocapsules », étant donné leur taille.

Par ailleurs, les zones de déclenchement déformables 7 et les logements 12 sont espacés les uns des autres d'une distance au moins égale à de l'ordre d'au moins la dimension d'une capsule de fragrance 13, notamment plus d'une fois et demie et jusqu'à deux à trois fois.

Les capsules de fragrance 13 du diffuseur 1 ne sont pas disposées au hasard proches les unes des autres dans des emplacements déterminés et identifiables, distants les uns des autres.

S'agissant d'un diffuseur 1 pour plusieurs utilisations comportant plusieurs capsules de fragrance 13, il peut être prévu soit qu'elles comportent toutes la même fragrance soit que certaines d'entre elles comportent des fragrances différentes. Dans ce dernier cas, il peut être prévu une plaque intérieure 6 formée de parties élémentaires juxtaposées avec des barrières intermédiaires imperméables aux fragrances.

Dans la réalisation représentée, dans laquelle le diffuseur 1 pour plusieurs utilisations présente une longueur de l'ordre de 8,5 cm, une largeur de l'ordre de 5,5 cm, et une épaisseur hors tout de l'ordre de 0,5 cm, il peut être prévu dix capsules de fragrance 13.

Plus généralement, dans le contexte de l'invention, la pluralité de zones de déclenchement déformables 7 par paroi extérieure 2a, 2b, de logements 12, de capsules de fragrance 13, est comprise entre deux et une vingtaine au maximum, plus particulièrement entre cinq et quinze, plus particulièrement encore de l'ordre de dix.

La plaque intérieure 6 du diffuseur 1 a, en l'absence de toute sollicitation de compression, une épaisseur (comptée en direction transversale Q) qui est légèrement plus petite que la dimension en direction transversale d'une capsule de fragrance 13, par exemple, plus petite de l'ordre du millimètre ou d'une fraction de millimètre. Par suite, une capsule de fragrance 13 saille légèrement des deux faces 11 de la plaque intérieure 6, ou du moins de l'une d'elles.

Complémentairement, chacune des deux parois extérieures perméables 2a, 2b, bien que globalement plate, comporte pour un diffuseur 1 pour plusieurs utilisations, une pluralité de zones conformées 8, chacune en forme générale de calotte sphérique à concavité tournée vers l'intérieur du diffuseur 1. Une telle zone conformée 8 saille de la face extérieure 3a de la paroi extérieure 2a, 2b. Elle est de forme globalement complémentaire de la partie de la capsule de fragrance 13 ayant son intégrité, qui saille de la plaque intérieure 6 et qui est au contact de la face intérieure 3b de la paroi extérieure 2a, 2b. Une telle zone conformée 8 est située au droit (dans la direction Q) d'un logement 12 (donc aussi d'une capsule de fragrance 13) et d'une zone de déclenchement déformable 7. Le diffuseur 1 comporte alors, pour une paroi extérieure 2a, 2b, autant de zones conformées 8 que de zones de déclenchement déformables 7. Une zone conformée 8 et une zone de déclenchement déformable 7 sont pour partie au moins communes, voire sont confondues, une zone conformée 8 se rapportant à la face intérieure 3b d'une paroi extérieure 2a, 2b, tandis qu'une zone de déclenchement déformable 7 se rapporte à sa face extérieure 3a. Dans cette réalisation, il est prévu pour chaque capsule de fragrance 13, deux zones conformées 8, de part et d'autre en direction transversale Q, chacune sur l'une des parois extérieures 2a, 2b.

Complémentairement, chacune des deux parois extérieures perméables 2a, 2b (ou du moins celle des parois extérieures 2a, 2b qui est perméable si les deux ne le sont pas), bien que globalement plate, comporte pour un diffuseur 1 pour plusieurs utilisations, une pluralité de zones conformées 8, chacune en forme générale de calotte

Etant possible que dans une réalisation une seule des deux parois 2a, 2b soit perméable à la fragrance, il est alors envisageable que seule la paroi extérieure perméable 2a, 2b, comporte des zones conformées 8, tandis que la paroi imperméable à la fragrance, 2b, 2a, ne comporte pas de zones conformées telles que celles précédemment décrites.

Dans la réalisation d'un diffuseur 1 pour une seule utilisation, il est prévu une seule zone conformée 8 par paroi extérieure perméable 2a, 2b.

Avec le diffuseur 1 précédemment décrit, une zone conformée 8 saille de la face extérieure 3a de la paroi 2a, 2b de l'ordre du millimètre ou de quelques millimètres.

Avec une paroi extérieure 2a, 2b en papier ou en carton, une zone conformée peut être réalisée par embossage. Le cas échéant, l'on réalise un amincissement de la paroi 2a, 2b.

Dans une réalisation possible, le diffuseur 1 pour plusieurs utilisations comporte vers, ou sur, la face extérieure 3a d'au moins une paroi extérieure 2a, 2b, une pluralité de moyens de repérage 16 de la localisation des zones de déclenchement déformables 7, afin que l'utilisateur sache où exercer la pression de déclenchement p.

En particulier un moyen de repérage 16 est sur la face extérieure 3a de la paroi extérieure 2a, 2b, comportant une zone de déclenchement déformable 7.

Un tel moyen de repérage 16 peut être ou comprendre la saillie sur la face extérieure 3a de la paroi 2a, 2b concernée, constituée par la zone conformée 8 correspondante. Le cas échéant, le moyen de repérage 16 peut aussi inclure un signe visible. Il peut aussi inclure une indication de la nature de la fragrance, surtout si le diffuseur 1 peut en diffuser plusieurs différentes.

Dans une réalisation, la ou les zones de déclenchement déformables 7, le ou les logements 12, la ou les capsules de fragrance 13, la ou les zones conformées 8, le ou les moyens de repérage 16 sont disposés de sorte à être écartés du champ périphérique du diffuseur 1 (pouvant notamment correspondre au champ périphérique de la plaque intérieure 6), par un espace marginal 14a.

Selon une réalisation, le diffuseur 1 comporte en outre au moins une partie 17 d'un moyen de suspension tel qu'un trou traversant apte à recevoir un lien d'accrochage. Ce trou 17 est situé dans l'espace marginal 14a ou dans un espace 14, afin de ne pas interférer avec une capsule de fragrance 13.

Dans une autre réalisation, il peut être envisagé que le diffuseur 1 soit fixé à un support par collage temporaire. Dans ce cas, une seulement des deux parois 2a, 2b est perméable à la fragrance, tandis que l'autre paroi 2b, 2a, est imperméable (et d'ailleurs ne comporte pas de zones conformées). Sur la face extérieure 3a, sans saillie, de cette paroi imperméable, il peut être prévu une couche adhésive double face, protégée avant fixation, par une pellicule arrachable.

Un diffuseur 1 tel qu'il vient d'être décrit peut être présenté à l'unité, ou bien il peut être prévu une plaquette de diffuseurs, comportant plusieurs diffuseurs 1 disposés côte à côte et séparés par des lignes frangibles. Cette plaquette peut être présentée à plat ou bien elle peut être repliée sur elle-même en accordéon.

Pour fabriquer un diffuseur 1 pour plusieurs usages, tel qu'il a été décrit, on a à disposition deux parois extérieures 2a, 2b ou la pièce 14, une plaque intérieure 6, et des capsules de fragrance 13.

Puis (flèche A de la figure 1), on dispose la plaque intérieure 6 sur la face intérieure 3b d'une première paroi extérieure 2a ou 2b, en regard de cette première paroi extérieure 2a ou 2b. On les solidarise.

Puis, alors que les capsules de fragrance 13, ayant leur intégrité, sont reçues dans les logements 12 de la plaque intérieure 6 (flèche B de la figure 1), on dispose la face intérieure 3b d'une seconde paroi extérieure 2b ou 2a, sur la plaque intérieure 6, en regard de celle-ci (flèche C de la figure 1). On les solidarise.

Selon les cas, on dispose les capsules de fragrance 13 sur la plaque intérieure 6 avant qu'elle soit disposée sur la face intérieure 3b d'une première paroi extérieure 2a ou 2b, ou bien on dispose les capsules de fragrance 13 sur la plaque intérieure 6 après qu'elle ait été disposée sur la face intérieure 3b d'une première paroi extérieure 2a ou 2b.

Comme déjà indiqué, si dans une application particulière, la substance volatile est une fragrance, il peut s'agir d'une substance différente mais analogue au regard de sa diffusion, par exemple une substance ayant des propriétés insecticides ou répulsives d'animaux (tels que mites ou moustiques), cette liste n'étant pas limitative.

## Revendications

1. Diffuseur (1) de substance volatile atmosphérique, d'intérieur, comportant :
- deux parois extérieures (2a, 2b), ayant chacune une face extérieure (3a) et une face intérieure (3b), espacées l'une en regard de l'autre, parallèlement,
∘ une étant perméable à la substance volatile, en direction transversale, de part en part,
∘ une pouvant, dans au moins une zone de déclenchement déformable (7), être déformée en direction transversale et dans le sens extérieur-intérieur consécutivement à une pression de déclenchement, suffisante, de mêmes direction et sens, exercée manuellement par l'utilisateur du diffuseur (1) pour déclencher la diffusion,
- une plaque intérieure (6) disposée entre les deux parois extérieures (2a, 2b), en regard d'elles, adjacente à la face intérieure (3b) de la paroi extérieure perméable (2a, 2b), formant avec les deux parois extérieures (2a, 2b) un ensemble solidaire limitée par deux faces (11),
∘ pourvue d'interstices (10) communicants entre eux et ouvrants sur ces faces (11), et compressible en direction transversale,
∘ ménageant en elle au moins un logement (12) au droit d'une zone de déclenchement déformable (7), recevant et maintenant en position une capsule de substance volatile (13),
- au moins une capsule de substance volatile (13), unitaire ou formée de l'agrégat de plusieurs capsules élémentaires,
∘ qui, ayant son intégrité, enferme une substance volatile, et est adjacente à la plaque intérieure (6) et à la face intérieure (3b) de la paroi extérieure perméable (2a, 2b),
∘ qui est apte à être rompue moyennant une pression transversale suffisante correspondant à la pression de déclenchement exercée par l'utilisateur, en libérant ainsi la substance volatile,
de sorte que :
- avant mise en oeuvre, la capsule de substance volatile (13) ait son intégrité,
- pour déclencher la diffusion de la substance volatile d'une capsule de substance volatile (13), et donc la mise en oeuvre du diffuseur (1), l'utilisateur exerce la pression de déclenchement sur la zone de déclenchement déformable (7) correspondant à la capsule (13), ce qui rompt cette capsule (13) et libère la substance volatile qui diffuse vers la face intérieure (3b) de la paroi extérieure perméable (2a, 2b), d'une part directement, d'autre part indirectement via les interstices (10) communicants entre eux et sur les faces (11) de la plaque intérieure (6), puis diffuse à travers la paroi extérieure perméable (2a, 2b) et ensuite dans l'atmosphère environnante.

2. Diffuseur (1) selon la revendication 1, **caractérisé en ce que**, avant toute première utilisation, ni les parois extérieures (2a, 2b) ni la plaque intérieure (6) ne sont imprégnées de la substance volatile, celle-ci étant enfermée dans les capsules de substance volatile (13).

3. Diffuseur (1) selon l'une des revendications 1 et 2, qui comporte une pluralité de zones de déclenchement déformables (7) analogues, une pluralité de logements (12) analogues, une pluralité de capsules de substance volatile (13) de dimensions analogues, reçues et maintenues en position dans la pluralité de logements (12), et des espaces (14) entre les zones de déclenchement déformables (7) et entre les logements (12) dans lesquels il n'y a pas de capsule de substance volatile (13), les capsules de substance volatile (13) étant distantes et séparées les unes des autres de sorte qu'une capsule de substance volatile (13) peut être rompue sans que soient rompues toutes les autres capsules de substance volatile (13) du diffuseur (1), l'utilisateur ayant le choix de déclencher la diffusion d'une capsule de substance volatile (13) sans déclencher celle de toutes les autres capsules de substance volatile (13), le diffuseur (1) permettant ainsi plusieurs utilisations distinctes, consécutives ou espacées.

4. Diffuseur (1) selon la revendication 3, dans lequel une capsule de substance volatile (13) a une dimension supérieure à 3 mm, notamment de l'ordre de 5 mm, et dans lequel les zones de déclenchement déformables (7) et les logements (12) sont espacés les uns des autres d'une distance au moins égale à de l'ordre de plus de deux à trois fois la dimension d'une capsule de substance volatile (13).

5. Diffuseur (1) selon l'une des revendications 1 à 4, qui comporte sur la face extérieure (3a) d'au moins une paroi extérieure (2a, 2b), notamment la paroi extérieure (2a, 2b) comportant au moins une zone de déclenchement déformable (7), au moins un moyen de repérage (16) de la localisation de ladite au moins une zone de déclenchement déformable (7).

6. Diffuseur (1) selon l'une des revendications 1 à 5, dans lequel la plaque intérieure (6) est réalisée sous la forme de matière plastique en mousse à cellules ouvertes, ou sous la forme de non-tissé, ou moyennant une structure ayant pour effet de retarder le temps mis par la substance volatile pour migrer de la capsule une fois rompue à la paroi extérieure perméable.

7. Diffuseur (1) selon l'une des revendications 1 à 6, dans lequel la plaque intérieure (6) a, en l'absence de toute sollicitation de compression, une épaisseur plus petite que la dimension d'une capsule de substance volatile (13).

8. Diffuseur (1) selon l'une des revendications 1 à 7, dans lequel la plaque intérieure (6) est adjacente aux deux faces intérieures des parois extérieures (2a, 2b).

9. Diffuseur (1) selon l'une des revendications 1 à 8, dans lequel la plaque intérieure (6) est au contact de la face intérieure (3b) de la paroi extérieure perméable (2a, 2b).

10. Diffuseur (1) selon l'une des revendications 1 à 9, dans lequel la plaque intérieure (6) est compressible élastiquement.

11. Diffuseur (1) selon l'une des revendications 1 à 10, dans lequel les deux parois extérieures (2a, 2b) et la plaque intérieure (6) forment un ensemble solidaire par collage.

12. Diffuseur (1) selon l'une des revendications 1 à 11, dans lequel un logement (12) de capsule est un espace vide, débouchant par une ouverture (12a) vers une paroi extérieure perméable (2a, 2b), dont la dimension dans le plan de la plaque intérieure (6) est ajustée à la dimension de la capsule de substance volatile (13) dans ce même plan, de sorte que la capsule de substance volatile (13) soit maintenue en position.

13. Diffuseur (1) selon l'une des revendications 1 à 12, dans lequel les deux parois extérieures (2a, 2b) sont deux parties d'une même pièce (4) pliée à l'endroit de deux rainages (5a, 5b), une portion du champ périphérique de la plaque intérieure (6) étant recouverte par une troisième partie (2c) de la pièce (4) entre les deux rainages (5a, 5b).

14. Diffuseur (1) selon l'une des revendications 1 à 13, dans lequel le champ périphérique (11a) de la plaque intérieure (6) est soit libre et exposé à l'atmosphère environnante, soit pour partie recouvert, soit totalement recouvert.

15. Diffuseur (1) selon l'une des revendications 1 à 14, dans lequel une face extérieure (3a) au moins des parois extérieures (2a, 2b) comporte une impression, des lettres, chiffres, motifs.

16. Diffuseur (1) selon l'une des revendications 1 à 15, qui comporte en outre au moins une partie (17) d'un moyen de suspension, notamment un trou traversant apte à recevoir un lien d'accrochage.

17. Diffuseur (1) selon l'une des revendications 1 à 16, **caractérisé en ce que** la substance volatile est une fragrance ; le diffuseur étant un diffuseur de fragrance.

18. Plaquette comportant plusieurs diffuseurs (1) de substance volatile atmosphérique, d'intérieur, selon l'une des revendications 1 à 17, disposés côte à côte et séparés par des lignes frangibles.

19. Procédé de fabrication d'un diffuseur (1) de substance volatile atmosphérique, d'intérieur, selon l'une des revendications 1 à 17, dans lequel :
- on a à disposition deux parois extérieures (2a, 2b) ou une pièce (4) pourvue de rainages (5a, 5b) permettant de réaliser les deux parois extérieures (2a, 2b),
- on a à disposition une plaque intérieure (6) pourvue d'interstices (10) communicants entre eux et ouvrants sur les faces (11) de la plaque intérieure (6), compressible en direction transversale, et ménageant en elle au moins un logement (12) apte à recevoir et maintenir en position une capsule de substance volatile (13),
- on a à disposition au moins une capsule de substance volatile (13), unitaire ou formée de l'agrégat de plusieurs capsules élémentaires,
- on dispose la plaque intérieure (6) sur la face intérieure (3b) d'une première paroi extérieure (2a, 2b), en regard de cette première paroi extérieure (2a, 2b) et on les solidarise,
- alors qu'au moins une capsule de substance volatile (13) est reçue dans le au moins un logement (12) de la plaque intérieure (6), on dispose la face intérieure (3b) d'une seconde paroi extérieure (2b, 2a) sur la plaque intérieure (6), en regard de la plaque intérieure (6) et on les solidarise.

## Patentansprüche

1. Diffusor (1) für flüchtige atmosphärische Stoffe, welcher folgendes umfasst:
- zwei Außenwände (2a, 2b), die jeweils eine Außenfläche (3a) und eine Innenfläche (3b) haben und parallel zueinander beabstandet sind,
∘ wobei eine für den flüchtigen Stoff in Querrichtung von einer Seite zur anderen durchlässig ist,
∘ und eine, die in zumindest einem formändernden Freisetzungsbereich (7) in Querrichtung von außen nach innen durch manuell vom Benutzer des Diffusors (1) in gleicher Richtung ausgeübten Freisetzungsdruck, der stark genug ist, um die Diffusion auszulösen, verformt werden kann,
- eine Innenplatte (6), die zwischen den beiden Außenwänden (2a, 2b) ihnen gegenüberliegend an die Innenfläche (3b) der durchlässigen Außenwand (2a, 2b) angrenzend angeordnet ist und mit den beiden Außenwänden (2a, 2b) eine feste Einheit bildet, die durch zwei Flächen (11) begrenzt wird,
∘ die versehen ist mit Zwischenräumen, die miteinander verbunden sind, sich zu diesen Flächen (11) hin öffnen und in Querrichtung zusammendrückbar sind,
∘ die in sich zumindest eine Aufnahme (12) senkrecht zu einem formändernden Freisetzungsbereich (7) ausbilden, die eine Kapsel mit flüchtigem Stoff (13) aufnimmt und in Position hält,
- zumindest eine Kapsel mit flüchtigem Stoff (13), die aus einer Kapsel besteht oder durch die Zusammenfassung mehrerer elementarer Kapseln gebildet wird,
∘ die in unversehrtem Zustand einen flüchtigen Stoff umschließt und an die Innenplatte (6) und an die Innenfläche (3b) der durchlässigen Außenwand (2a, 2b) angrenzt,
∘ die in der Lage ist, durch einen ausreichend starken, quer einwirkenden Druck, der dem vom Benutzer ausgeübten Freisetzungsdruck entspricht, gebrochen zu werden und dadurch den flüchtigen Stoff freisetzt, so dass:
- die Kapsel mit dem flüchtigen Stoff (13) vor der Benutzung unversehrt ist,
- zum Auslösen der Verbreitung des flüchtigen Stoffs einer Kapsel mit flüchtigem Stoff (13), und folglich vor der Benutzung des Diffusors (1) der Benutzer den entsprechenden Freisetzungsdruck auf den formändernden Freisetzungsbereich (7) ausübt, wodurch die Kapsel (13) bricht und der flüchtige Stoff zur Innenseite (3b) der durchlässigen Außenwand (2a, 2b) hin zum einen direkt, zum anderen indirekt über die an den Flächen (11) der Innenplatte (6) miteinander verbundenen Zwischenräume (10), dann durch die durchlässige Außenwand (2a, 2b) hindurch anschließend in der Umgebung verbreitet wird.

2. Diffusor (1) nach Anspruch 1, **dadurch gekennzeichnet, dass** vor jeder Erstbenutzung weder die Außenwände (2a, 2b) noch die Innenplatte (6) mit dem flüchtigen Stoff imprägniert sind, da dieser zwischen den Kapseln mit flüchtigem Stoff (13) eingeschlossen ist.

3. Diffusor (1) nach einem der Ansprüche 1 und 2, der eine Vielzahl ähnlicher formändernder Freisetzungsbereiche (7), eine Vielzahl ähnlicher Aufnahmen (12), eine Vielzahl ähnlicher Kapseln mit flüchtigem Stoff (13) mit ähnlichen Abmessungen umfasst, die in der Vielzahl an Aufnahmen (12) und in den Räumen (14) zwischen den formändernden Freisetzungsbereichen (7) und zwischen den Aufnahmen (12), in welchen sich keine Kapsel mit flüchtigem Stoff (13) befindet, aufgenommen und in Position gehalten werden, wobei die Kapseln mit flüchtigem Stoff (13) zueinander beabstandet und voneinander getrennt werden, sodass eine Kapsel mit flüchtigem Stoff (13) gebrochen werden kann, ohne dass die anderen Kapseln mit flüchtigem Stoff (13) des Diffusors (1) gebrochen werden, wobei der Benutzer die Möglichkeit hat, bei einer Kapsel mit flüchtigem Stoff (13) eine Verteilung auszulösen, ohne dies bei den anderen Kapseln mit flüchtigem Stoff (13) zu tun, wobei der Diffusor (1) somit mehrere unterschiedliche Nutzungen hintereinander oder in Abständen ermöglicht.

4. Diffusor (1) nach Anspruch 3, in welchem eine Kapsel mit flüchtigem Stoff (13) eine Abmessung größer 3 mm, insbesondere aber von 5 mm hat, und in welcher die formändernden Freisetzungsbereiche und die Aufnahmen (12) einen Abstand von zumindest zwei bis dreimal die Abmessung einer Kapsel mit flüchtigem Stoff (13) zueinander aufweisen.

5. Diffusor (1) nach einem der Ansprüche 1 bis 4, welcher an der Außenfläche (3a) von zumindest einer Außenwand (2a, 2b), insbesondere der Außenwand (2a, 2b), welche zumindest einen formändernden Freisetzungsbereich (7) umfasst, zumindest ein Mittel zur Markierung (16) der Stelle des besagten, zumindest einen formändernden Freisetzungsbereichs (7) umfasst.

6. Diffusor (1) nach einem der Ansprüche 1 bis 5, in welchen die Innenplatte (6) in Form eines Kunststoff-Schaumstoffes mit offenen Zellen oder in Form von Non-Woven hergestellt wird oder mittels einer Struktur, welche die Dauer für das Austreten des flüchtigen Stoffs aus der Kapsel nach dem Brechen der durchlässigen Außenwand verzögert.

7. Diffusor (1) nach einem der Ansprüche 1 bis 6, in welchen die Innenplatte (6) bei Nichtbeanspruchung durch Zusammendrücken eine Dicke aufweist, die kleiner ist, als die Abmessung einer Kapsel mit flüchtigem Stoff (13).

8. Diffusor (1) nach einem der Ansprüche 1 bis 7, in welchem die Innenplatte (6) an zwei Innenflächen der Außenwände (2a, 2b) angrenzt.

9. Diffusor (1) nach einem der Ansprüche 1 bis 8, in welchem die Innenplatte (6) mit der Innenfläche (3b) der durchlässigen Außenwand (2a, 2b) in Berührung ist.

10. Diffusor (1) nach einem der Ansprüche 1 bis 9, in welchem die Innenplatte (6) elastisch zusammendrückbar ist.

11. Diffusor (1) nach einem der Ansprüche 1 bis, 10, in welchem die beiden Außenwände (2a, 2b) und die Innenplatte (6) durch Verkleben eine feste Einheit bilden.

12. Diffusor (1) nach einem der Ansprüche 1 bis 11, in welchem eine Kapselaufnahme (12) ein leerer Raum ist, der durch eine Öffnung (12a) zu einer durchlässigen Außenwand (2a, 2b) führt, deren Abmessung auf der Ebene der Innenplatte (6) an die Abmessung der Kapsel mit flüchtigem Stoff (13) auf dieser Ebene angepasst ist, sodass die Kapsel mit flüchtigem Stoff (13) in Position gehalten wird.

13. Diffusor (1) nach einem der Ansprüche 1 bis 12, in dem die beiden Außenwände (2a, 2b) zwei Teile ein und desselben Stücks (4) sind, welches an der Stelle der zwei Falze (5a, 5b) geknickt wird, wobei ein Abschnitt des umlaufenden Feldes der Innenplatte (6) von einen dritten Teil (2c) des Stücks (4) zwischen den beiden Falzen (5a, 5b) bedeckt wird.

14. Diffusor (1) nach einem der Ansprüche 1 bis 13, in welchen das umlaufende Feld (11a) der Innenplatte (6) entweder frei oder der Umgebungsatmosphäre ausgesetzt ist oder im bedeckten Teil vollkommen bedeckt ist.

15. Diffusor (1) nach einem der Ansprüche 1 bis 14, in welchem zumindest eine Außenfläche (3a) der Außenwände (2a, 2b) einen Druck, Buchstaben, Ziffern, Motive umfasst.

16. Diffusor (1) nach einem der Ansprüche 1 bis 15, der des Weiteren zumindest einen Teil (17) eines Aufhängemittels umfasst, insbesondere eine Durchgangsbohrung zur Aufnahme eines Aufhängebands.

17. Diffusor (1) nach einem der Ansprüche 1 bis 16, **dadurch gekennzeichnet, dass** der flüchtige Stoff ein Duftstoff ist, da der Diffusor ein Duftdiffusor ist.

18. Platte mit mehreren Innendiffusoren (1) eines flüchtigen atmosphärischen Stoffs nach einem der Ansprüche 1 bis 17, die durch durchbrechbare Linien voneinander getrennt nebeneinander angeordnet sind.

19. Verfahren zur Herstellung eines Innendiffusors (1) eines flüchtigen atmosphärischen Stoffs, nach einem der Ansprüche 1 bis 17, in welchem:
- man zwei Außenwände (2a, 2b) zur Verfügung hat oder ein Stück (4), welches mit Falzen (5a, 5b) versehen ist und das Herstellen der zwei Außenwände (2a, 2b) ermöglicht,
- man eine Innenplatte (6) mit Zwischenräumen (10) zur Verfügung hat, die miteinander verbunden sind und sich zu den Flächen (11) der Innenplatte (6) hin öffnen, in Querrichtung zusammendruckbar ist und in sich zumindest eine Aufnahme (12) ausbildet, die eine Kapsel mit flüchtigem Stoff (13) aufnehmen und in Position halten kann,
- man zumindest eine Kapsel mit flüchtigem Stoff (13) zur Verfügung hat, die aus einer Kapsel besteht oder durch die Zusammenfassung mehrerer elementarer Kapseln gebildet wird,
- man die Innenplatte (6) auf der Innenfläche (3b) einer ersten Außenwand (2a, 2b) gegenüber dieser ersten Außenwand (2a, 2b) anordnet und man sie miteinander verbindet,
- man, während zumindest eine Kapsel mit flüchtigem Stoff (13) in die zumindest eine Aufnahme (12) der Innenplatte (6) aufgenommen wird, die Innenfläche (3b) einer zweiten Außenwand (2b, 2a) auf die Innenplatte (6) gegenüber der Innenplatte (6) anordnet und sie verbindet.

## Claims

1. An inner volatile atmospheric substance diffuser (1) which comprises :
- two outer walls (2a, 2b), each having an outer (3a) face and an inner (3b) face, which are spaced apart and mutually opposite and parallel to each other,
• one face of which being pervious to the volatile substance, in the transversal direction, from side to side,
• one face of which, in at least one shape-changing release area (7), can change shape in the transversal direction, and the outside-inside direction, further to a sufficient release pressure along the same direction and in the same orientation, manually exerted by the user of the diffuser (1) to activate the diffusion;
- an inner plate (6) positioned between and opposite the two outer walls (2a, 2b), adjacent to the inner face (3b) of the pervious outer wall (2a, 2b), and forming with the two outer walls (2a, 2b), a rigidly connected assembly limited by two faces (11),
• provided with gaps (10) communicating with each other and opening on such faces (11), and compressible in the transverse direction,
• and provided with a cavity (12) therein, above a shape-changing release area (7), which receives and holds a volatile substance capsule (13) in position,
- at least one volatile substance capsule (13) which, whether it is one unit or consists of a set of several elementary capsules,
• in the integrity thereof, contains a volatile substance and is adjacent to the inner plate (6) and to the inner face (3b) of the pervious outer wall (2a, 2b),
• which is breakable by means of a sufficient transversal pressure, which corresponds to the release pressure exerted by the user, thus releasing the volatile substance,
such that:
- before use, the volatile substance capsule (13) has its integrity,
- in order to trigger the diffusion of the volatile substance from a volatile substance capsule (13), and thus to activate the diffuser, the user exerts the release pressure onto the shape-changing release area (7) which corresponds to the capsule (13), thus breaking said capsule (13) and releasing the volatile substance that diffuses towards the inner face (3b) of the pervious outer wall (2a, 2b), directly on the one hand and indirectly on the other hand via the communicating gaps (10) and on the faces (11) of the inner plate (6), then diffuses through the pervious outer wall (2a, 2b) and then into the surrounding atmosphere.

2. A diffuser (1) according to claim 1, **characterized in that**, prior to any first use, neither the outer walls (2a, 2b) nor the inner plate (6) are impregnated with the volatile substance, the latter being contained in the volatile substance capsules (13).

3. A diffuser (1) according to one of claims 1 and 2, which comprises a plurality of analog shape-changing release areas (7), a plurality of analog cavities (12), a plurality of volatile substance capsules (13) having analog dimensions, received and held in position in the plurality of cavities (12), and spaces (14) between the shape-changing release areas (7) and between the cavities (12), wherein there is no volatile substance capsule (13), with the volatile substance capsules (13) being spaced and separated from each other so that a volatile substance capsule (13) can be broken without all the other ones of the volatile substance capsules (13) of the diffuser (1) being broken, with the user having the option of triggering the diffusion of a volatile substance capsule (13) without triggering that of all the other volatile substance capsules (13), with the diffuser (1) thus enabling several distinct uses, whether consecutive or spaced.

4. A diffuser (1) according to claim 3, wherein a volatile substance capsule (13) has a dimension greater than 3mm, in particular of the order of 5mm, and wherein the shape-changing release areas (7) and the cavities (12) are spaced from each other by a distance at least equal to more than two or three times the size of a volatile substance capsule (13).

5. A diffuser (1) according to one of claims 1 to 4, which includes, on the outer face (3a) of at least one outer wall (2a, 2b), specifically the outer wall (2a, 2b) including at least one shape-changing release area (7), at least one reference means (16) for locating said at least one shape-changing release area (7).

6. A diffuser (1) according to one of claims 1 to 5, wherein the inner plate (6) is made of open cell foam plastic material, or of nonwoven fabric, or with a structure having the effect of extending the time taken for the volatile material to migrate from the capsule once broken to the outer pervious wall.

7. A diffuser (1) according to one of claims 1 to 6, wherein the inner plate (6) has, in the absence of any compression load, a slightly smaller thickness than the dimension of a volatile substance capsule (13).

8. A diffuser (1) according to one of claims 1 to 7, wherein the inner plate (6) is adjacent to the two inner faces of the two outer walls (2a, 2b).

9. A diffuser (1) according to one of claims 1 to 8, wherein the inner plate (6) is in contact with the inner face (3b) of the pervious outer wall (2a, 2b).

10. A diffuser (1) according to one of claims 1 to 9, wherein the inner plate (6) is elastically compressible.

11. A diffuser (1) according to one of claims 1 to 10, wherein the two outer walls (2a, 2b) and the inner plate (6) form an assembly rigidly connected by gluing.

12. A diffuser (1) according to one of claims 1 to 11, wherein a cavity (12) for a capsule is an empty space, which leads to an opening (12a), towards a pervious outer wall (2a, 2b), the dimension of which, in the plane of the inner plate (6), is adjusted to the dimension of the volatile substance capsule (13) in the same plane, so that the volatile substance capsule (13) is held in position.

13. A diffuser (1) according to one of claims 1 to 12, wherein the two outer walls (2a, 2b) are two portions of a same piece (4) folded at two grooves (5a, 5b), with a portion of the peripheral field of the inner plate (6) being covered with a third portion (2c) of the piece (4) between the two grooves (5a, 5b).

14. A diffuser (1) according to one of claims 1 to 13, wherein the peripheral field (11a) of the inner plate (6) is either free and exposed to the surrounding atmosphere or is either partly covered, or totally covered.

15. A diffuser (1) according to one of claims 1 to 14, wherein at least one outer face (3a) of the outer walls (2a, 2b) comprises printing, letters, numbers, patterns.

16. A diffuser (1) according to one of claims 1 to 15, which further comprises at least one portion (17) of a suspension means, specifically a through hole adapted to receive a coupling link.

17. A diffuser (1) according to one of claims 1 to 16, **characterized in that** the volatile substance is a fragrance; with the diffuser then being a fragrance diffuser.

18. A platelet including several inner volatile atmospheric substance diffusers (1) according to one of claims 1 to 17, disposed side by side and separated by frangible lines.

19. A method for manufacturing an inner volatile atmospheric substance diffuser (1), according to one of claims 1 to 17, wherein:
- two outer walls (2a, 2b) or a piece (4) provided with grooves (5a, 5b) are/is provided for making the two outer walls (2a, 2b),
- an inner plate (6) is provided with gaps (10) communicating together and opening on the faces (11) of the inner plate, transversally compressible, and providing therein at least one cavity (12) adapted to receive and hold in position a volatile substance capsule (13),
- at least one volatile substance capsule (13) is provided, whether it is one unit or consists of a set of several elementary capsules,
- the inner plate (6) is provided on the inner face (3b) of a first outer wall (2a, 2b), opposite said first outer wall (2a, 2b) and is secured thereto,
- whereas at least one volatile substance capsule (13) is received in the at least one cavity (12) of the inner plate (6), the inner face (3b) of a second outer wall (2b, 2a) is positioned on the inner plate (6), opposite the inner plate (6) and is secured thereto.
